# EUROPÄISCHE PATENTANMELDUNG

(11) **EP 3 932 291 A1**
(43) Veröffentlichungstag der Anmeldung: **05.01.2022**
(21) Anmeldenummer: 21183065.8
(22) Anmeldetag: 01.07.2021
(51) Int. Cl.: A61B 3/00, A61B 3/135, A41D 13/11

(54) **SCHUTZANORDNUNG FÜR EIN MEDIZINISCHES UNTERSUCHUNGSGERÄT**

(30) Priorität: 02.07.2020 DE 102020208302
(71) Anmelder: Ucar, Ufuk, 75031 Eppingen (DE)
(72) Erfinder: Ucar, Ufuk, 75031 Eppingen (DE); Doll, Theo, 74193 Schwaigern (DE)
(74) Vertreter: Patentanwälte Ruff, Wilhelm, Beier, Dauster & Partner mbB

(57) **Zusammenfassung**

1. Schutzanordnung für ein medizinisches Untersuchungsgerät.
2.1 Eine derartige Schutzanordnung zur Trennung einer Bedienerseite von einer Patientenseite an dem Untersuchungsgerät ist bekannt.
2.2 Erfindungsgemäß ist ein Schutzflächenteil vorgesehen, das sich in montiertem Zustand am Untersuchungsgerät entlang einer Trennebene zwischen der Bedienerseite und der Patientenseite erstreckt, wobei das Schutzflächenteil wenigstens eine zentrale Aussparung aufweist, die auf einen Aufnahmeabschnitt des Untersuchungsgeräts für ein lösbares Funktionsteil des Untersuchungsgeräts derart abgestimmt ist, dass das Schutzflächenteil mit der Aussparung auf den Aufnahmeabschnitt aufsteckbar und gemeinsam mit einem Befestigen des gelösten Funktionsteils auf dem Aufnahmeabschnitt fixierbar ist.
2.3 Einsatz bei einem Spaltlampenmikroskop.

## Beschreibung

Die Erfindung betrifft eine Schutzanordnung für ein medizinisches Untersuchungsgerät zur Trennung einer Bedienerseite von einer Patientenseite an dem Untersuchungsgerät, mit einem formstabilen, zumindest teilweise transparenten Schutzflächenteil, das sich in montiertem Zustand am Untersuchungsgerät entlang einer Trennebene zwischen der Bedienerseite und der Patientenseite erstreckt.

Medizinische Untersuchungsgeräte sind für unterschiedliche medizinische Anwendungszwecke allgemein bekannt. Im Bereich der Augenheilkunde, die auch als Ophthalmologie bezeichnet wird, werden Spaltlampenmikroskope verwendet, um die Augen eines Patienten untersuchen zu können. Bei einem solchen Spaltlampenmikroskop, aber auch bei anderen medizinischen Untersuchungsgeräten, kommt die medizinisch geschulte Bedienperson einer Patientin oder einem Patienten zwangsläufig relativ nahe. Dabei kann eine Ansteckungsgefahr im Hinblick auf Mikroorganismen bestehen. Insbesondere unter dem Eindruck der COVID-19-Pandemie sind derartige Risiken zu beachten.

Aufgabe der Erfindung ist es, eine Schutzanordnung der eingangs genannten Art zu schaffen, die das Risiko einer Ansteckungsgefahr zwischen Bedienerseite und Patientenseite reduzieren kann.

Diese Aufgabe wird durch die Merkmale des Anspruchs 1 gelöst. Die erfindungsgemäße Schutzanordnung ist zur Trennung einer Bedienerseite von einer Patientenseite an dem Untersuchungsgerät vorgesehen und weist ein formstabiles, zumindest teilweise transparentes Schutzflächenteil auf, das sich in montiertem Zustand am Untersuchungsgerät entlang einer Trennebene zwischen der Bedienerseite und der Patientenseite erstreckt. Die zumindest teilweise transparente Gestaltung des Schutzflächenteils ermöglicht für beide Seiten, d.h. sowohl für die Bedienerseite als auch für die Patientenseite, den Blick auf die jeweils andere Seite durch das Schutzflächenteil hindurch. Das Schutzflächenteil weist wenigstens eine zentrale Aussparung auf, die auf einen Aufnahmeabschnitt des Untersuchungsgeräts für ein lösbares Funktionsteil des Untersuchungsgeräts derart abgestimmt ist, dass das Schutzflächenteil mit der Aussparung auf den Aufnahmeabschnitt aufsteckbar und gemeinsam mit einem Befestigen des gelösten Funktionsteils auf dem Aufnahmeabschnitt fixierbar ist. Erfindungsgemäß wird das Schutzflächenteil bei seinem Einsatz am Untersuchungsgerät direkt im Bereich eines Funktionsteils des Untersuchungsgeräts montiert, wobei in einfacher Weise das Funktionsteil entfernt, das Schutzflächenteil aufgesteckt und anschließend das Funktionsteil wieder befestigt wird, wodurch zwangsläufig auch die gleichzeitige Fixierung des Schutzflächenteils erfolgt. Die erfindungsgemäße Lösung eignet sich sowohl für stationär - insbesondere in Arztpraxen oder Kliniken - positionierte Untersuchungsgeräte als auch für bewegliche Untersuchungsgeräte oder für handgeführte Untersuchungsgeräte wie insbesondere Handrefraktometer in der Augenheilkunde. Dadurch, dass das Schutzflächenteil direkt an dem medizinischen Untersuchungsgerät befestigt wird, wobei in einfacher Weise ein lösbares Funktionsteil eine Doppelfunktion übernimmt, werden aufwändige Gestelle oder Halterungen vermieden, um das Schutzflächenteil zu positionieren. Die Doppelfunktion des Funktionsteils bestimmt sich zum einen durch seine Untersuchungsfunktion in montiertem Zustand am Untersuchungsgerät und zum anderen durch die Befestigungsfunktion für das Schutzflächenteil. Die erfindungsgemäße Schutzanordnung eignet sich für eine Vielzahl unterschiedlicher medizinischer Untersuchungsgeräte aus dem Bereich der Augenheilkunde, dem Bereich der Hals-Nasen-Ohrenheilkunde oder dem Bereich der Hautheilkunde (Dermatologie), oder aus anderen medizinischen Bereichen. Bedienpersonen auf der Bedienerseite können Ärztinnen oder Ärzte, medizinisches Personal oder auch Medizintechniker, wie insbesondere Augenoptikerinnen und -optiker, sein.

Die erfindungsgemäße Schutzanordnung eignet sich in besonders guter Weise als Schutz gegen die Übertragung von Mikroorganismen zwischen Patienten und Bediener, vorzugsweise als Schutz gegen eine Tröpfcheninfektion, da das Schutzflächenteil in montiertem Zustand am Untersuchungsgerät eine über eine Trennebene vollständig geschlossene Trennwand darstellt. Kleinste Tröpfchen in Aerosolen, die von der Bedienerseite oder der Patientenseite her in Richtung der jeweils anderen Seite fliegen, werden durch die Schutzanordnung wirksam abgehalten. Bei zusätzlicher antiseptischer Gestaltung des Außenteils und/oder des Innenteils des Schutzflächenteils ist zudem gewährleistet, dass entsprechende Mikroorganismen wie insbesondere Viren oder Bakterien, die über die Tröpfchen auf das Schutzflächenteil treffen, unschädlich gemacht werden. Die erfindungsgemäße Schutzanordnung eignet sich demzufolge in besonders guter Weise als Schutz gegen eine Ansteckung durch Bakterien oder Viren, insbesondere Corona-Viren.

In Ausgestaltung der Erfindung weist das Schutzflächenteil ein transparentes Innenteil mit der Aussparung sowie ein insbesondere nichttransparentes Außenteil auf, wobei wenigstens das Außenteil wenigstens einseitig eine antimikrobiell wirksame Oberflächenschicht aufweist. Das Außenteil kann ein- oder mehrstückig gestaltet sein. Insbesondere weist das Außenteil zwei miteinander verbundene Teilabschnitte auf, zwischen denen das Innenteil eingefügt ist. Vorteilhaft ist die antimikrobiell wirksame Oberflächenschicht beidseitig des Außenteils vorgesehen. Die antimikrobiell wirksame Oberflächenschicht ist dazu vorgesehen, Mikroorganismen wie insbesondere Bakterien oder Viren unschädlich zu machen. Die antimikrobiell wirksame Oberflächenschicht erstreckt sich vorzugsweise wenigstens auf einer Seite über eine gesamte Außenfläche des Außenteils. Die antimikrobiell wirksame Oberflächenschicht hat eine desinfizierende Wirkung. Zur Erzielung dieser desinfizierenden Wirkung weist die Oberflächenschicht vorzugsweise Silber- und/oder Kupferionen auf. Die antimikrobiell wirksame Oberflächenschicht kann durch Koextrusion oder durch Auflaminieren einer Kunststofffolie erzeugt werden, wobei entweder in dem koextrudierten Kunststoffträgermaterial oder in der Kunststofffolie die antimikrobiell wirksamen Mittel wie insbesondere Silber- und/oder Kupferionen eingebracht sind.

In weiterer Ausgestaltung der Erfindung sind das Innenteil und das Außenteil stoffschlüssig miteinander verbunden. Der Stoffschluss wird insbesondere durch Verklebung oder durch Verschweißung erzielt.

In weiterer Ausgestaltung der Erfindung ist das Außenteil aus einem thermoplastischen Kunststoffmaterial und das Innenteil aus einem transparenten Kunststoffmaterial oder aus Glas hergestellt. Falls das Innenteil aus einem transparenten Kunststoffmaterial hergestellt ist, ist es vorzugsweise aus Polycarbonat gestaltet.

In weiterer Ausgestaltung der Erfindung ist dem transparenten Innenteil eine auf eine Oberfläche des Innenteils gerichtete Lichtstrahleinrichtung zugeordnet, die in Funktion zur Inaktivierung von auf der Oberfläche befindlichen Mikroorganismen ausgestaltet ist. Die Lichtstrahleinrichtung erzeugt somit eine Inaktivierung von auf der Oberfläche des Innenteils befindlichen Mikroorganismen. Dadurch ist eine Desinfektion der Oberfläche des Innenteils durch Lichtstrahlung ermöglicht. Die Lichtstrahleinrichtung kann von dem Schutzflächenteil getrennt oder mit dem Schutzflächenteil verbunden sein. Vorzugsweise ist die Lichtstrahleinrichtung an dem Schutzflächenteil angeordnet, so dass das Schutzflächenteil und die Lichtstrahleinrichtung als Einheit gemeinsam handhabbar sind.

In weiterer Ausgestaltung der Erfindung weist die Lichtstrahleinrichtung wenigstens ein UVC-Leuchtmittel auf. Die UVC-Lichtstrahlung ist besonders gut geeignet, um Mikroorganismen wie insbesondere Viren und damit auch Corona-Viren vom Typ SARS-CoV-2 zu schädigen und zu inaktivieren.

In weiterer Ausgestaltung der Erfindung ist zur Aufnahme des UVC-Leuchtmittels ein Gehäuse vorgesehen, das einstückig an dem Außenteil angeformt ist. Das Gehäuse für das UVC-Leuchtmittel wird somit bei der Herstellung des Außenteils aus einem thermoplastischen Kunststoffmaterial zwangsläufig mit hergestellt. Zusätzliche Montagevorgänge zur Befestigung des Gehäuses an dem Außenteil werden hierdurch vermieden.

In weiterer Ausgestaltung der Erfindung ist das Außenteil zumindest abschnittsweise haubenartig gestaltet mit wenigstens einem Flächenabschnitt, der zu der Bedienerseite und/oder zu der Patientenseite hin gewölbt ist. Der den haubenartigen Abschnitt bildende Flächenabschnitt erstreckt sich somit aus der Trennebene heraus und umgrenzt die Bedienerseite und/oder die Patientenseite zumindest abschnittsweise. Hierdurch wird ein Schutz gegen die Ausbreitung von Mikroorganismen, insbesondere von Viren, weiter verbessert, da auch dieser haubenartige Flächenabschnitt als Teilbereich des Außenteils mit der antimikrobiell wirksamen Oberflächenschicht versehen ist. Vorzugsweise ist dieser Flächenabschnitt dreidimensional gewölbt. Vorteilhaft sind zwei haubenartige Flächenabschnitte vorgesehen, von denen einer sich zu der Patientenseite und der andere zu der Benutzerseite hin erstrecken. Die beiden haubenartigen Flächenabschnitte können einstückige Fortsätze von jeweils einem Teilabschnitt des Außenteiles sein, zwischen denen das Innenteil eingefügt ist.

In weiterer Ausgestaltung der Erfindung ist das Schutzflächenteil im Bereich einer Unterseite mit einer nach unten offenen, ergonomischen Durchgriffsöffnung versehen, die ein Hindurchgreifen von der Bedienerseite zu der Patientenseite ermöglicht. Dadurch ist gewährleistet, dass die Bedienperson auch Verstellelemente an dem medizinischen Untersuchungsgerät erreichen kann, die auf der Patientenseite des Untersuchungsgeräts angeordnet sind. Die Durchgriffsöffnung kann am Außenteil oder am Innenteil vorgesehen sein. Diese Ausgestaltung ist insbesondere vorteilhaft bei stationären medizinischen Untersuchungsgeräten, vorzugsweise bei einem Spaltlampenmikroskop.

In weiterer Ausgestaltung der Erfindung ist dem Schutzflächenteil eine formschlüssig wirksame Verdrehsicherung zugeordnet, die in montiertem Zustand des Schutzflächenteils eine verdrehsichere Fixierung auf dem Aufnahmeabschnitt ermöglicht. Diese Ausgestaltung ist vorteilhaft dann vorgesehen, wenn die Aussparung des Schutzflächenteils rotationssymmetrisch ausgeführt ist. Durch die Verdrehsicherung wird gewährleistet, dass das Schutzflächenteil sich im Betrieb des Untersuchungsgeräts nicht unbeabsichtigt verdreht, weil das Schutzflächenteil durch die Patientenseite oder die Bedienerseite berührt wird.

In weiterer Ausgestaltung der Erfindung weist das Schutzflächenteil patientenseitig wenigstens eine optisch signifikante Markierung auf, die eine Patientin/ein Patient optisch fokussieren kann. Diese Ausgestaltung ist besonders vorteilhaft einsetzbar bei einer Schutzanordnung für ein Spaltlampenmikroskop, da eine Patientin oder ein Patient die Augen auf diese optisch signifikante Markierung richten kann, wodurch sich eine definierte Augenausrichtung des Patienten oder der Patientin ergibt, die der Bedienperson die Untersuchung des jeweiligen Auges erleichtert. Vorzugsweise sind mehrere, über die der Patientenseite zugewandte Seite des Schutzflächenteils verteilt angeordnete Markierungen vorgesehen, die eine Fokussierung der Augen der Patientin oder des Patienten in unterschiedlichen Richtungen ermöglichen. Derartige Markierungen können einfache Symbole oder auch Figuren sein. Figuren, insbesondere in Form von Comicfiguren, sind besonders gut geeignet, um die Aufmerksamkeit der Augen von Kindern zu erlangen.

In weiterer Ausgestaltung der Erfindung ist dem Schutzflächenteil eine Absaugeinrichtung zugeordnet, die zur Absaugung von Aerosolen im Bereich des Schutzflächenteils ausgebildet ist. Die Absaugeinrichtung ist vorzugsweise der Patientenseite zugewandt, um durch die Patientenseite ausgestoßene Aerosole ansaugen und so die Bedienerseite schützen zu können. Es ist auch möglich, die Absaugeinrichtung auf beiden Seiten des Schutzflächenteiles vorzusehen, d. h. sowohl auf der Bedienerseite als auch auf der Patientenseite.

In weiterer Ausgestaltung der Erfindung weist die Absaugeinrichtung wenigstens eine entlang des Schutzflächenteils verlegte Schlauch- oder Rohreinheit auf, der ein Saugantrieb zugeordnet ist. Als Saugantrieb ist vorzugsweise eine elektrische Pumpe vorgesehen. Der Schlauch- oder Rohreinheit und/oder dem Saugantrieb kann zusätzlich eine Abführeinrichtung zugeordnet sein, die die durch die Schlauch- oder Rohreinheit abgesaugte Luft entweder in ein luftdichtes Behältnis oder aber in eine Umgebung abführt, die außerhalb eines Raumes befindlich ist, in dem die Schutzanordnung zum Einsatz kommt. Vorzugsweise ist die Abführeinrichtung so gestaltet, dass die abgesaugte Luft und damit die abgesaugten Aerosole über eine Gebäudeöffnung, insbesondere ein Fenster, in eine Außenumgebung des Gebäudes abgeleitet werden. Die Schlauch- oder Rohreinheit ist der Bedienerseite oder der Patientenseite zugeordnet und in entsprechender Weise an dem Schutzflächenteil verlegt. Falls zwei Schlauch- oder Rohreinheiten vorgesehen sind, können diese sowohl der Patientenseite als auch der Bedienerseite des Schutzflächenteiles zugeordnet und entsprechend gegenüberliegend verlegt sein.

In weiterer Ausgestaltung der Erfindung weist die Schlauch- oder Rohreinheit mehrere über eine Länge der Schlauch- oder Rohreinheit verteilt angeordnete Saugöffnungen auf. Die Saugöffnungen können als einfache, in die Schlauch- oder Rohreinheit eingebrachte Löcher gestaltet sein. Alternativ oder ergänzend können die Saugöffnungen mit Strömungsleitelementen wie insbesondere Ansaugdüsen versehen sein.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt schematisch in einer Seitenansicht eine Ausführungsform einer erfindungsgemäßen Schutzanordnung für ein medizinisches Untersuchungsgerät in Form eines lediglich schematisch dargestellten Spaltlampenmikroskops,
- Fig. 2: die Schutzanordnung nach Fig. 1 in perspektivischer Darstellung von einer Patientenseite her,
- Fig. 3: die Schutzanordnung nach den Fig. 1 und 2 in perspektivischer Darstellung von einer Bedienerseite her,
- Fig. 4: in perspektivischer Darstellung eine weitere Ausführungsform einer erfindungsgemäßen Schutzanordnung für ein lediglich schematisch dargestelltes medizinisches Untersuchungsgerät,
- Fig. 5: ausschnittsweise einen Längsschnitt durch die Schutzanordnung gemäß Fig. 4,
- Fig. 6: die Schutzanordnung nach den Fig. 4 und 5 in perspektivischer Darstellung von einer Patientenseite her,
- Fig. 7: in perspektivischer Darstellung die Schutzanordnung nach den Fig. 4 bis 6 schräg von unten und von einer Bedienerseite her,
- Fig. 8: eine weitere Ausführungsform einer erfindungsgemäßen Schutzanordnung für ein medizinisches Untersuchungsgerät in Form eines lediglich schematisch dargestellten Spaltlampenmikroskops,
- Fig. 9: eine schematische Schnittdarstellung der Schutzanordnung gemäß Fig. 8 und
- Fig. 10: in perspektivischer Darstellung die Schutzanordnung nach den Fig. 8 und 9 unter Weglassung der schematischen Darstellung des medizinischen Untersuchungsgerätes.

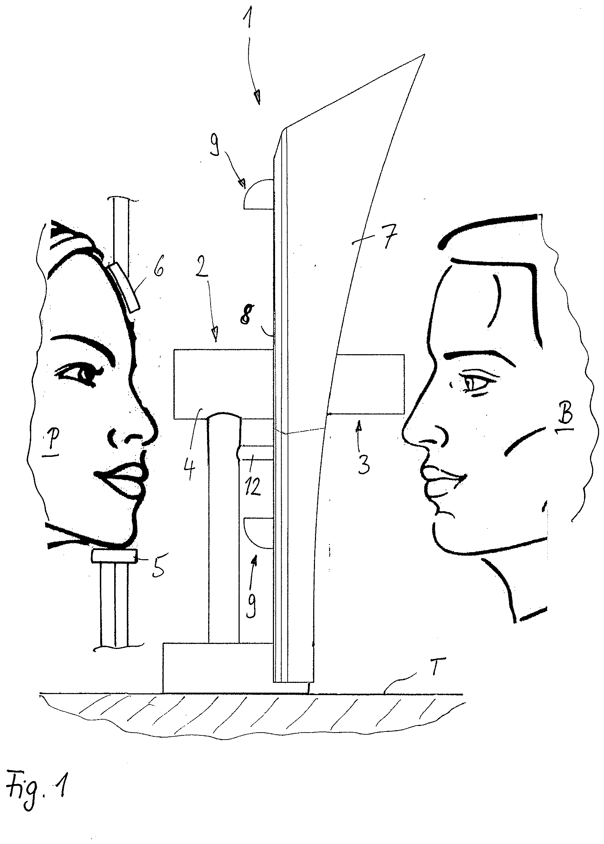

Eine Schutzanordnung nach den Fig. 1 bis 3 weist ein Schutzflächenteil 1 auf, das in der schematischen Darstellung gemäß Fig. 1 in nachfolgend näher beschriebener Weise an einem medizinischen Untersuchungsgerät 2, vorliegend in Form eines Spaltlampenmikroskops, montiert ist. Das Untersuchungsgerät 2 weist ein Gestell auf, das auf einer Tischfläche T aufsteht. Das Untersuchungsgerät 2 ist auf einer Bedienerseite B mit einem Funktionsteil in Form eines Okulars 3 versehen. Auf einer Patientenseite P ist das Untersuchungsgerät 2 mit Stützen 5 und 6 versehen, über die ein Kopf einer Patientin oder eines Patienten kinn- und stirnseitig vor dem Untersuchungsgerät 2 abstützbar ist.

Das Untersuchungsgerät 2 weist - ebenfalls patientenseitig - eine Okularaufnahme 4 auf, mit der das Okular 3 lösbar verbunden ist, sobald das Untersuchungsgerät 2 an der Okularaufnahme 4 befestigt ist.

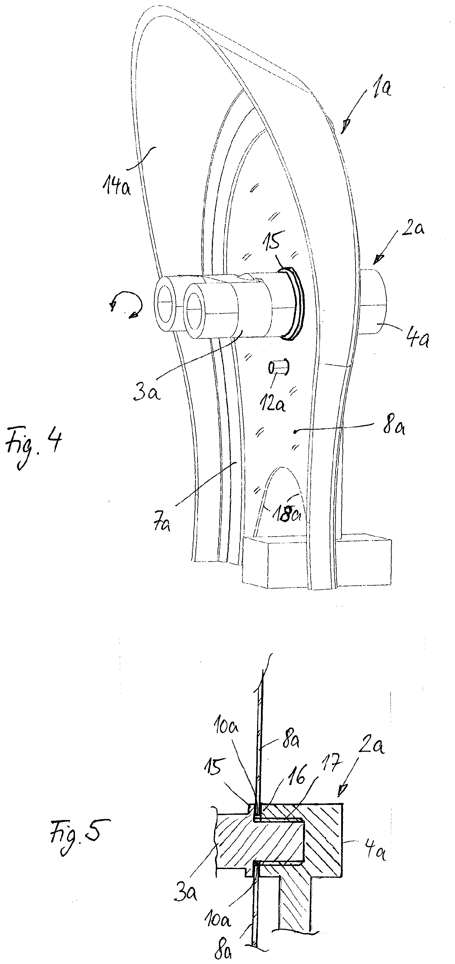

Die Schutzanordnung weist ein Schutzflächenteil 1 auf, das in der Darstellung gemäß Fig. 1 an dem Untersuchungsgerät 2 befestigt ist. Hierfür wird zunächst das Okular 3 von der Okularaufnahme 4 gelöst und entfernt. Anschließend wird das Schutzflächenteil 1 mit einer nachfolgend näher beschriebenen, zentralen Aussparung 10 auf eine Stirnseite der Okularaufnahme 4 aufgesteckt, wobei die Aussparung 10 an einer ringförmigen Stirnfläche der Okularaufnahme 4 anschlägt. Anschließend wird das Okular 3 wieder aufgeschraubt, wodurch zwangsläufig das Schutzflächenteil 1 im Bereich der zentralen Aussparung 10 zwischen der Okularaufnahme 4 und dem Okular 3 festgeklemmt wird. Um zu vermeiden, dass das Schutzflächenteil 1 sich aufgrund seiner rotationssymmetrischen Aussparung 10 um einen zylindrischen Aufnahmeabschnitt der Okularaufnahme 4 dreht, ist zusätzlich eine Verdrehsicherung vorgesehen, die durch ein kleines Loch 11 im Schutzflächenteil 1 unterhalb der zentralen Aussparung 10 und durch einen Sicherungsstift 12 andererseits erzielt wird, der an dem Gestell des Untersuchungsgeräts 2 befestigt ist und in montiertem Zustand des Schutzflächenteils 1 durch das Loch 11 hindurchragt. Die Befestigung des Schutzflächenteils 1 zwischen der Okularaufnahme 4 und dem Okular 3 entspricht im Wesentlichen einer Befestigung, wie sie in dem weiteren Ausführungsbeispiel gemäß den Fig. 4 bis 7 anhand der Fig. 5 schematisch dargestellt ist. Im Gegensatz zu der Darstellung gemäß Fig. 5 ist bei der Ausführungsform gemäß den Fig. 1 bis 3 vorgesehen, dass ein Aufnahmeabschnitt der Okularaufnahme 4 zylindrisch in Richtung des Okulars 3 abragt und mit einem Außengewinde versehen ist, auf den ein Innengewinde des Okulars 3 aufgeschraubt wird.

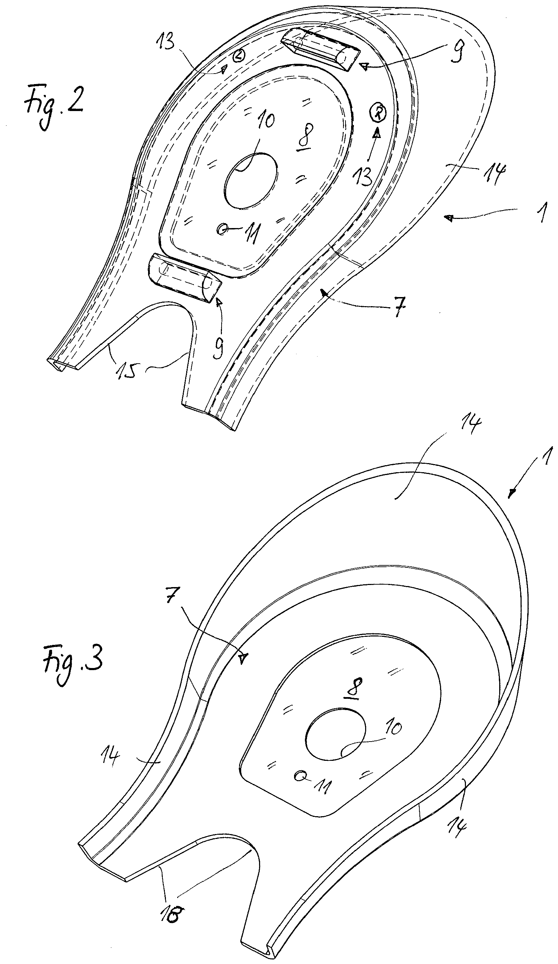

Wie anhand der Fig. 2 und 3 erkennbar ist, wird das Schutzflächenteil 1 durch ein Außenteil 7 sowie durch ein Innenteil 8 gebildet, wobei das Innenteil 8 an seinem umlaufenden Randbereich durchgängig stoffschlüssig mit einem Auflagerand des Außenteils 7 verbunden ist. Vorliegend ist das Innenteil 8 über eine umlaufende Verklebung mit dem Außenteil 7 verbunden. Das Innenteil 8 ist als transparente Scheibe ausgeführt, die aus Glas oder einem transparenten Kunststoffmaterial wie insbesondere Polycarbonat hergestellt ist. Das transparente Innenteil 8 ist mit der zentralen Aussparung 10 und dem Loch 11 für die Verdrehsicherung versehen. Das Innenteil 8 wird umlaufend umgeben von dem Außenteil 7, das als einschaliges, thermoplastisches Kunststoffbauteil ausgeführt ist. Das Außenteil 7 weist einen flächigen Trennbereich auf, der sich etwa in einer Ebene des Innenteils 8 erstreckt und somit gemeinsam mit dem Innenteil 8 eine Trennebene des Schutzflächenteils 1 bildet. An diesen Trennabschnitt schließt einstückig ein gewölbter Flächenabschnitt 14 an, der von einem Randbereich des Trennabschnitts in Richtung der Bedienerseite B winklig abragt, wie Fig. 3 oder Fig. 4 gut zu entnehmen ist. Der Flächenabschnitt 14 bildet eine Haubenform. Das Außenteil 7 ist zudem unterhalb des Ausschnitts, in den das Innenteil 8 eingesetzt ist, mit einer ergonomischen Durchgriffsöffnung 18 versehen, die sich zu einem unteren Randbereich des Außenteils 7 hin öffnet, wie den Fig. 2 und 3 gut zu entnehmen ist.

Dem transparenten Innenteil 8 sind ober- und unterseitig zwei Lichtstrahleinrichtungen 9 zugeordnet, die jeweils ein zu der Patientenseite P hin abragendes, einstückig aus dem Außenteil 7 ausgeformtes Gehäuse aufweisen, das jeweils umlaufend geschlossen und lediglich auf seiner zu dem Innenteil 8 gewandten Seite offen ist. In jedem Gehäuse der Lichtstrahleinrichtung 9 ist ein UVC-Leuchtmittel montiert. Das jeweilige Gehäuse bildet einen Reflektor für das jeweilige UVC-Leuchtmittel, so dass UVC-Lichtstrahlung im Betrieb der UVC-Leuchtmittel von unten bzw. von oben her auf die Oberfläche des transparenten Innenteils 8 auf der der Patientenseite P zugewandten Seite des Schutzflächenteils 1 abgestrahlt wird.

Das nichttransparente Außenteil 7 ist über seine gesamte, der Patientenseite P zugewandte Außenfläche mit einer antimikrobiell wirksamen Oberflächenschicht versehen, die auf der Oberfläche des Außenteils 7 befindliche Mikroorganismen unschädlich macht. Die Oberflächenschicht ist mit Silber- und/oder Kupferionen versehen.

Die Aussparung 10 ist beim dargestellten Ausführungsbeispiel kreisrund gestaltet und weist einen Durchmesser auf, der lediglich geringfügig größer ist als ein Durchmesser eines Aufnahmeabschnitts der Okularaufnahme 4, so dass die Aussparung 10 zusammen mit dem Innenteil 8 und dem Außenteil 7 weitgehend spielfrei koaxial zu einer Aufsteckachse des Aufnahmeabschnitts der Okularaufnahme 4 aufsteckbar ist. Die Aussparung 10 ist zudem so bemessen, dass ihr Durchmesser geringer ist als ein Durchmesser einer Stirnfläche des Okulars 3, die beim Aufschrauben des Okulars 3 auf einen Randbereich der Aussparung 10 auf der Bedienerseite B auftrifft und den Randbereich der Aussparung 10 relativ zu der Okularaufnahme 4 festklemmt. Im montierten Zustand des Schutzflächenteils 1 zwischen dem Okular 3 und der Okularaufnahme 4 verbleibt somit kein Spalt im Bereich der Aussparung 10. Vielmehr sind die umlaufenden Randbereiche der Aussparung 10 sowohl auf Patientenseite P als auch auf Bedienerseite B über die entsprechenden Stirnflächen des Aufnahmeabschnitts der Okularaufnahme 4 einerseits und des Okulars 3 andererseits dicht abgeschlossen. Im Bereich des Lochs 11 und des Sicherungsstifts 12 kann in nicht näher dargestellter Weise zusätzlich eine Ringdichtung vorgesehen sein, um auch im Bereich des Lochs 11 einen dichten Abschluss mit dem Sicherungsstift 12 zu erzielen.

Wie anhand der Fig. 2 erkennbar ist, weist das Außenteil 7 auf der Patientenseite P zwei Markierungen 13 auf, die mit "L" und "R" für "links" und "rechts" gekennzeichnet sind. Diese Markierungen 13 dienen dazu, dass eine Patientin oder ein Patient auf der Patientenseite die Augen in eine entsprechende Richtung fokussieren kann. Dies ist vor allem vorteilhaft, wenn das medizinische Untersuchungsgerät 2 ein Spaltlampenmikroskop ist, bei dem während der Untersuchung die Augen der Patienten oder des Patienten in unterschiedlichen Richtungen zeitweise verharren müssen, damit die Bedienerseite einen Augenhintergrund gut inspizieren kann. Derartige Markierungen sind besonders vorteilhaft bei Kindern als Patientinnen oder Patienten, da diese Markierungen 13 einen Blickfang für die Kinder bilden können. Besonders vorteilhaft ist es dann, wenn die Markierungen als für Kinder interessante Symbole wie insbesondere Comicfiguren oder Ähnliches gestaltet sind. Die Markierungen 13 sind entweder nachträglich auf das Schutzflächenteil 1 aufgebracht oder bei der Herstellung des Innenteils 8 oder des Außenteils 7 einstückig mit ausgeformt.

Die Schutzanordnung gemäß den Fig. 4 bis 7 entspricht vom grundsätzlichen Aufbau und einer grundsätzlichen Befestigung an einem medizinischen Untersuchungsgerät 2a der Schutzanordnung gemäß den Fig. 1 bis 3, so dass zur Vermeidung von Wiederholungen ergänzend auf die Beschreibung und die Zeichnungen zur Ausführungsform gemäß den Fig. 1 bis 3 verwiesen wird. Funktionsgleiche Bauteile und Abschnitte der Schutzanordnung gemäß den Fig. 4 bis 7 sind mit gleichen Bezugszeichen versehen wie die Schutzanordnung gemäß den Fig. 1 bis 3, jedoch unter Hinzufügung des Buchstabens a.

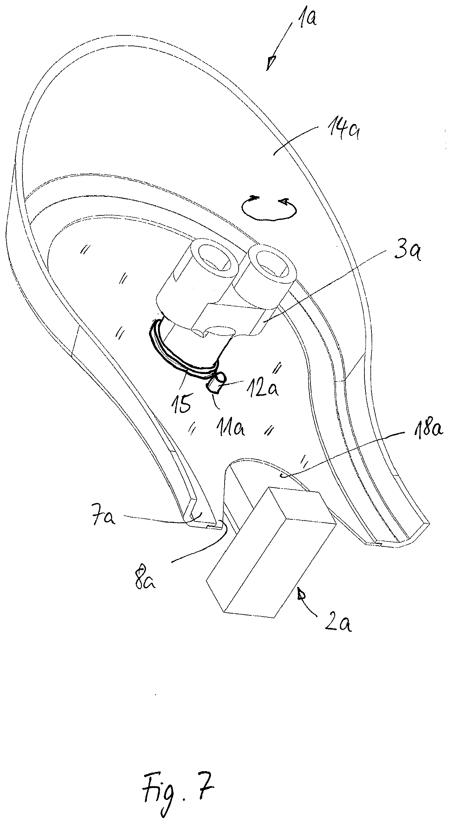

Das medizinische Untersuchungsgerät 2a ist ebenfalls als stationäres Gerät ausgeführt, das auf einem Untergrund wie insbesondere einer Tischfläche in funktionsbereitem Zustand fest aufsteht. Auch das Untersuchungsgerät 2a weist ein Okular 3a und eine Okularaufnahme 4a auf, wobei die Okularaufnahme 4a über ein nicht näher bezeichnetes Gestell stationär abgestützt ist. Das Okular 3a ist, wie die Pfeilrichtungen in den Fig. 4 und 7 erkennen lassen, mit der Okularaufnahme 4a verschraubbar oder von dieser lösbar. Hierzu weist das Okular 3a einen koaxial abragenden Schraubstutzen mit einem Außengewinde auf, der in eine komplementäre Schraubaussparung 17 der Okularaufnahme 4a einschraubbar ist. Die Okularaufnahme 4a weist, wie Fig. 5 entnommen werden kann, eine ringförmige Stirnfläche 16 auf, an der das Innenteil 8a mit seiner kreisförmigen Aussparung 10a axial abgestützt ist. Das Innenteil 8a mit der Aussparung 10a ist auf den Schraubstutzen des Okulars 3a aufgesteckt und stützt sich axial an einem umlaufenden Ringbund 15 des Okulars 3a ab, so dass die Aussparung 10a mit ihrem umlaufenden Randbereich zwischen der Stirnfläche 16 und dem Ringbund 15 in montiertem Zustand des Okulars 3a eingeklemmt ist.

Das Schutzflächenteil 1a der Schutzanordnung gemäß den Fig. 4 bis 7 setzt sich ebenfalls aus einem transparenten Innenteil 8a und einem nichttransparenten Außenteil 7a zusammen. Das Innenteil 8a ist als Polycarbonatscheibe ausgeführt und erstreckt sich bis zu einem unteren Rand des Außenteils 7a. Das Außenteil 7a umschließt demzufolge das Innenteil 8a nicht umlaufend, sondern lediglich im Seiten- und Kopfbereich des Innenteils 8a. Das Innenteil 8a ist mit der Durchgriffsöffnung 18a versehen, die bei dem Schutzflächenteil 1 gemäß den Fig. 1 bis 3 im Außenteil vorgesehen ist. Die Durchgriffsöffnung 18a ist in gleicher Weise zu einem unteren Randbereich des Innenteils 8a hin offen, wie dies bei der Durchgriffsöffnung 18 gemäß den Fig. 2 und 3 der Fall ist.

Das Innenteil 8a ist als Polycarbonatscheibe ausgeführt und in seinem Randbereich flächig mit dem Außenteil 7a verklebt. Hierzu weist das Außenteil 7a, wie Fig. 7 zu entnehmen ist, einen umlaufenden Randsteg auf, auf dem der Randbereich des Innenteils 8a so bündig aufliegt, dass eine der Patientenseite P zugewandte Seite des Innenteils 8a bündig mit der entsprechenden Oberfläche des Außenteils 7a abschließt. Das Außenteil 7a ist analog der Ausführungsform gemäß den Fig. 1 bis 3 als einschaliges thermoplastisches Kunststoffbauteil gestaltet. Das Außenteil 7a ist beim dargestellten Ausführungsbeispiel nicht transparent und ist beidseitig, d.h. sowohl auf der Patientenseite als auch auf der Bedienerseite, mit einer antimikrobiell wirksamen Oberflächenschicht versehen.

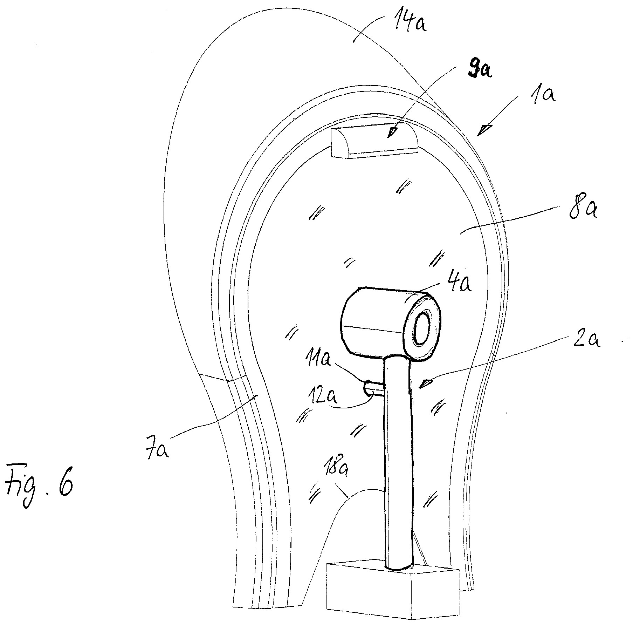

Wie anhand der Fig. 6 erkennbar ist, ist auf der Patientenseite dem transparenten Innenteil 8 analog zur Ausführungsform nach den Fig. 1 bis 3 ebenfalls eine Lichtstrahleinrichtung 9a zugeordnet, die dazu vorgesehen ist, auf einer Oberfläche des Innenteils 8a befindliche Mikroorganismen zu inaktivieren. Die Lichtstrahleinrichtung 9a weist zum einen ein Gehäuse auf, das umlaufend geschlossen, jedoch in Richtung der Oberfläche des Innenteils 8a offen ist. Zum anderen weist die Lichtstrahleinrichtung 9a ein UVC-Leuchtmittel auf, das in dem Gehäuse angeordnet ist. Das Gehäuse kann einstückig aus dem Außenteil 7a ausgeformt oder als separates Bauteil hergestellt und auf dem Außenteil 7a oberhalb des Innenteils 8a befestigt sein. Das Gehäuse wirkt als Reflektor für die Lichtstrahlung des UVC-Leuchtmittels, so dass UVC-Lichtstrahlen von der Lichtstrahleinrichtung 9a ausgehend auf die der Patientenseite P zugewandte Oberfläche des Innenteils 8a abgestrahlt werden.

Das Außenteil 7a ist analog der Ausführungsform nach den Fig. 1 bis 3 zusätzlich zu dem das Innenteil 8a umgebenden Trennabschnitt mit einem in Richtung der Bedienerseite B winklig abragenden, haubenförmigen Flächenabschnitt 14a versehen, der gemeinsam mit dem Trennabschnitt des Außenteils 7a einstückig hergestellt ist.

Bei der Ausführungsform gemäß den Fig. 4 bis 7 ist ein Loch 11a in gleicher Weise unterhalb der zentralen Aussparung 10a vorhanden, wie dies bei der Ausführungsform gemäß den Fig. 1 bis 3 der Fall ist. Auch ein Sicherungsstift 12a, der in montiertem Zustand das Loch 11a durchdringt, weist die gleiche Funktion auf wie der Sicherungsstift 12 gemäß der Ausführungsform nach den Fig. 1 bis 3.

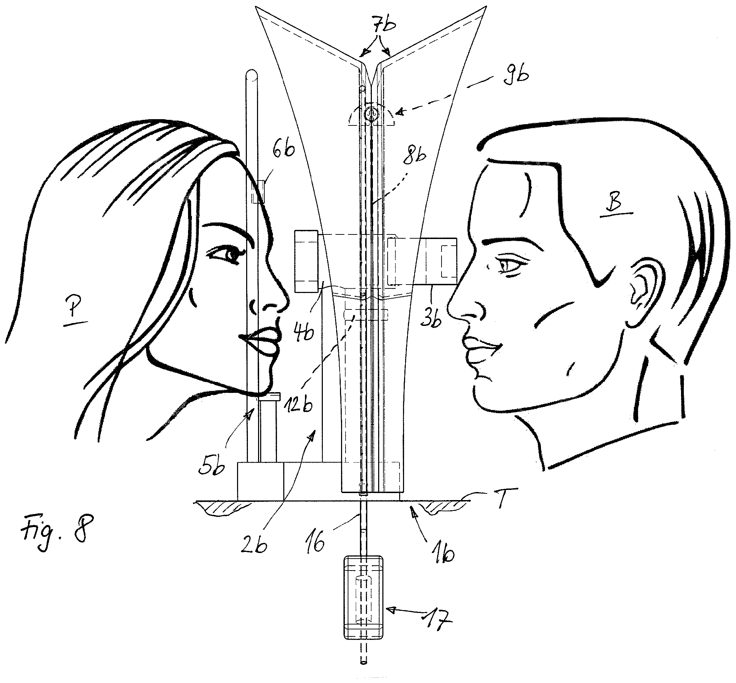

Die Schutzanordnung gemäß den Fig. 8 bis 10 ist in gleicher Weise für ein medizinisches Untersuchungsgerät 2b in Form eines Spaltlampenmikroskops vorgesehen wie die Schutzanordnung nach einer der zuvor beschriebenen Ausführungsformen. Vom grundsätzlichen Aufbau und von einer grundsätzlichen Befestigung an dem medizinischen Untersuchungsgerät 2b entspricht die Schutzanordnung den zuvor beschriebenen Ausführungsformen gemäß den Fig. 1 bis 7. Zur Vermeidung von Wiederholungen wird daher ergänzend auf die Offenbarung zu den beiden Ausführungsformen nach den Fig. 1 bis 7 verwiesen. Funktionsgleiche Bauteile und Abschnitte der Schutzanordnung gemäß den Fig. 8 bis 10 weisen die gleichen Bezugszeichen auf wie die Schutzanordnungen gemäß den Fig. 1 bis 7, jedoch unter Hinzufügung des Buchstabens b. Die verwendeten Materialien entsprechen den Materialien, wie sie anhand der Fig. 1 bis 7 bereits beschrieben wurden.

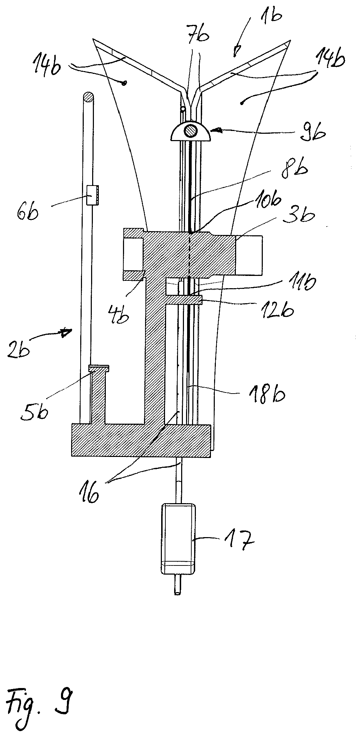

Das medizinische Untersuchungsgerät 2b bildet analog den zuvor beschriebenen Ausführungen ein stationäres Gerät, das in funktionsbereitem Zustand auf einer Tischfläche T oder einem anderen Untergrund fest aufsteht. Das Untersuchungsgerät 2b weist in gleicher Weise ein Okular 3b und eine Okularaufnahme 4b auf, wie dies bei den zuvor beschriebenen Ausführungsformen der Fall ist. Auch bei dem Untersuchungsgerät 2b nach den Fig. 8 und 9 ist das Okular 3b lösbar mit der Okularaufnahme 4b verschraubt. Die lösbare Verschraubung des Okulars 3b relativ zur stationären Okularaufnahme 4b ist anhand der Fig. 8 und 9 lediglich angedeutet.

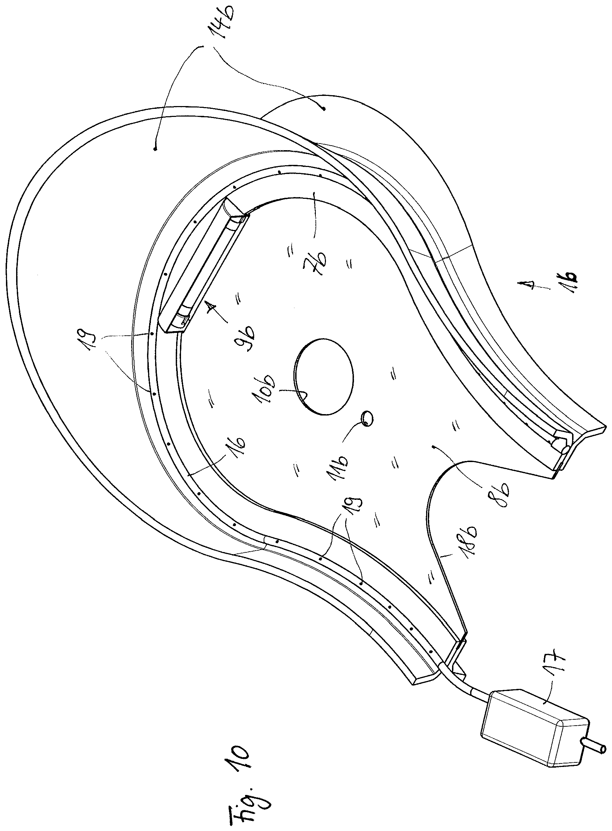

Die Schutzanordnung nach den Fig. 8 bis 10 weist ein Schutzflächenteil 1b auf, das sich aus einem transparenten Innenteil 8b und einem nicht transparenten Außenteil 7b zusammensetzt. Das nicht transparente Außenteil 7b wird durch zwei Teilabschnitte gebildet, die jeweils einstückig mit je einem haubenförmigen Flächenabschnitt 14b versehen sind. Die haubenförmigen Flächenabschnitte 14b ragen entgegengerichtet zueinander ab, so dass ein Flächenabschnitt 14b zur Patientenseite P und der andere Flächenabschnitt 14b zur Bedienerseite B abragt. Das transparente, scheibenförmige Innenteil 8b ist zwischen die beiden Teilabschnitte des Außenteiles 7b stoffschlüssig eingefügt, wie den Fig. 9 und 10 gut entnehmbar ist. Die beiden Teilabschnitte des Außenteiles 7b sind ebenfalls an ihrem Verbindungsbereich stoffschlüssig, insbesondere durch Verklebung, miteinander verbunden.

Das Innenteil 8b weist analog den zuvor beschriebenen Ausführungsformen eine zentrale, kreisförmige Aussparung 10b auf, die zur Montage des Schutzflächenteiles 1b auf der Okularaufnahme 4b und zur Fixierung mittels des aufschraubbaren Okulars 3b vorgesehen ist. In Abstand unterhalb der Aussparung 10b ist ein Loch 11b vorgesehen, das gemeinsam mit dem Sicherungsstift 12b des medizinischen Untersuchungsgeräts 2b die Verdrehsicherung für das Schutzflächenteil 1b ergibt, wie dies bei den zuvor beschriebenen Ausführungsformen ebenfalls vorgesehen ist. Das Innenteil 8b weist entsprechend den Ausführungsformen nach den Fig. 4 bis 7 an einem unteren Endbereich die ergonomische Durchgriffsöffnung 18b auf.

Zur Unschädlichmachung von Mikroorganismen an den gegenüberliegenden Scheibenflächen des Innenteils 8b ist bei der Ausführungsform nach den Fig. 8 bis 10 eine Lichtstrahleinrichtung 9b vorgesehen, die beide Scheibenflächen und damit sowohl die der Patientenseite P als auch die der Bedienerseite B zugewandte Scheibenfläche des Innenteils 8b bestrahlt, um entsprechende Mikroorganismen unschädlich zu machen. Die Besonderheit bei dem Schutzflächenteil 1b ist es, dass die Lichtstrahleinrichtung 9b oberhalb des Innenteils 8b in einer nicht näher bezeichneten Halteaufnahme des Außenteiles 7b so positioniert ist, dass ein in einem entsprechenden Gehäuse der Lichtstrahleinrichtung 9b untergebrachtes UVC-Leuchtmittel beide Scheibenflächen des Innenteiles 8b gleichzeitig bestrahlen kann. Das Gehäuse weist hierzu einen Reflektor auf, der sich, wie anhand der Fig. 9 erkennbar ist, durch die beiden Teilabschnitte des Außenteiles 7b hindurch erstreckt und das zentral in einer Mittelebene des Schutzflächenteils 1b angeordnete UVC-Leuchtmittel der Lichtstrahleinrichtung 9b derart umgibt, dass UVC-Lichtstrahlung des UVC-Leuchtmittels auf beiden Seiten des Innenteiles 8b nach unten in Richtung der jeweiligen Scheibenfläche des Innenteiles 8b abstrahlt. Somit wird lediglich eine einzelne Lichtstrahleinrichtung 9b vorgesehen, um sowohl die der Patientenseite P als auch die der Bedienerseite B zugewandte Scheibenfläche des Innenteiles 8b zu bestrahlen.

Der Patientenseite P des Schutzflächenteiles 1b ist zudem eine Absaugeinrichtung zugeordnet, die eine an dem der Patientenseite P zugewandten Teilabschnitt des Außenteiles 7b verlegte Schlaucheinheit 16 umfasst. Die Schlaucheinheit 16 erstreckt sich umlaufend um das Innenteil 8b herum und folgt einer Kontur des haubenförmigen Flächenabschnittes 14b. Die Schlaucheinheit 16 ist in nicht näher dargestellter Weise an dem Außenteil 7b positionsgesichert. Wie anhand der Fig. 10 erkennbar ist, ist die Schlaucheinheit 16 mit einer Vielzahl von gleichmäßig über die Länge der Schlaucheinheit 16 verteilt angeordneten Saugöffnungen 19 versehen. Die Schlaucheinheit 19 ist zudem mit einem Saugantrieb 17 versehen, der eine elektrische Pumpe aufweist. Der Saugantrieb 17 ist über einen nicht näher bezeichneten Anschlussstutzen mit einer Abführeinrichtung verbunden, die eine Abführung der über die Saugöffnungen 19 angesaugten Luft vornimmt. Die Abführeinrichtung kann mit einer geeigneten Filtereinrichtung für Mikroorganismen versehen sein, die die Mikroorganismen unschädlich macht, die mit der Luft im Bereich der der Patientenseite P zugewandten Scheibenfläche des Innenteiles 8b abgesaugt werden.

Die Absaugeinrichtung dient dazu, Aerosole, die sich durch Atmung oder Sprechen einer Person auf der Patientenseite P ergeben, abzusaugen. Die Lichtstrahleinrichtung 9b ist ergänzend vorgesehen, um sich an den Scheibenflächen niederschlagende Mikrotröpfchen von schwereren Aerosolen zu bestrahlen und entsprechende Mikroorganismen, insbesondere Viren, unschädlich zu machen. Die der Abführeinrichtung zugeordnete Filtereinrichtung kann als UV-Filtereinrichtung ausgeführt sein.

Das Außenteil 7b und da insbesondere die Teilabschnitte des Außenteiles 7b und ihre Flächenabschnitte 14b sind mit einer antimikrobiell wirksamen Oberflächenschicht versehen. Diese führt dazu, dass sich im Bereich des Außenteiles 7b absetzende Mikroorganismen durch die entsprechende Oberflächenschicht unschädlich gemacht werden. Die Ansaugeinrichtung mit der Schlaucheinheit 16 und den Ansaugöffnungen 19 sowie dem Saugantrieb 17 saugt zudem zumindest im Bereich der Patientenseite P Luft und demzufolge Aerosole ab, die weitere schädliche Mikroorganismen wie insbesondere Viren enthalten können. Die Schutzanordnung gemäß den Fig. 8 bis 10 bietet demzufolge einen wirkungsvollen Schutz gegen die Ansteckung von Personen wie insbesondere Patientinnen und Patienten oder medizinischem Personal mit schädlichen Bakterien oder Viren. Damit ist die Schutzanordnung besonders gut geeignet für medizinische Untersuchungen während einer Corona-Pandemie.

## Patentansprüche

1. Schutzanordnung für ein medizinisches Untersuchungsgerät (2, 2a, 2b) zur Trennung einer Bedienerseite (B) von einer Patientenseite (P) an dem Untersuchungsgerät (2, 2a, 2b), mit einem formstabilen, zumindest teilweise transparenten Schutzflächenteil (1, 1a, 1b), das sich in montiertem Zustand am Untersuchungsgerät (2, 2a, 2b) entlang einer Trennebene zwischen der Bedienerseite (B) und der Patientenseite (P) erstreckt, wobei das Schutzflächenteil (1, 1a, 1b) wenigstens eine zentrale Aussparung (10, 10a, 10b) aufweist, die auf einen Aufnahmeabschnitt des Untersuchungsgeräts (2, 2a) für ein lösbares Funktionsteil des Untersuchungsgeräts (2, 2a, 2b) derart abgestimmt ist, dass das Schutzflächenteil (1, 1a, 1b) mit der Aussparung (10, 10a, 10b) auf den Aufnahmeabschnitt aufsteckbar und gemeinsam mit einem Befestigen des gelösten Funktionsteils auf dem Aufnahmeabschnitt fixierbar ist.

2. Schutzanordnung nach Anspruch 1, **dadurch gekennzeichnet, dass** das Schutzflächenteil (1, 1a, 1b) ein transparentes Innenteil (8, 8a, 8b) mit der Aussparung (10, 10a, 10b) sowie ein insbesondere nichttransparentes Außenteil (7, 7a, 7b) aufweist, wobei wenigstens das Außenteil (7, 7a, 7b) wenigstens einseitig eine antimikrobiell wirksame Oberflächenschicht aufweist.

3. Schutzanordnung nach Anspruch 2, **dadurch gekennzeichnet, dass** das Innenteil (8, 8a, 8b) und das Außenteil (7, 7a, 7b) stoffschlüssig miteinander verbunden sind.

4. Schutzanordnung nach Anspruch 2 oder 3, **dadurch gekennzeichnet, dass** das Außenteil (7, 7a, 7b) aus einem thermoplastischen Kunststoffmaterial und das Innenteil (8, 8a, 8b) aus einem transparenten Kunststoffmaterial oder aus Glas hergestellt ist.

5. Schutzanordnung nach einem der vorhergehenden Ansprüche, **dadurch gekennzeichnet, dass** dem transparenten Innenteil (8, 8a, 8b) eine auf eine Oberfläche des Innenteils (8, 8a, 8b) gerichtete Lichtstrahleinrichtung (9, 9a, 9b) zugeordnet ist, die in Funktion zur Inaktivierung von auf der Oberfläche befindlichen Mikroorganismen ausgestaltet ist.

6. Schutzanordnung nach Anspruch 5, **dadurch gekennzeichnet, dass** die Lichtstrahleinrichtung (9, 9a, 9b) an dem Schutzflächenteil (1, 1a, 1b) angeordnet ist.

7. Schutzanordnung nach Anspruch 5 oder 6, **dadurch gekennzeichnet, dass** die Lichtstrahleinrichtung (9, 9a, 9b) wenigstens ein UVC-Leuchtmittel aufweist.

8. Schutzanordnung nach Anspruch 7, **dadurch gekennzeichnet, dass** zur Aufnahme des UVC-Leuchtmittels ein Gehäuse vorgesehen ist, das einstückig an dem Außenteil (7) ausgeformt ist.

9. Schutzanordnung nach einem der vorhergehenden Ansprüche, **dadurch gekennzeichnet, dass** das Außenteil (7, 7a, 7b) zumindest abschnittsweise haubenartig gestaltet ist mit wenigstens einem Flächenabschnitt (14, 14a, 14b), der zu der Bedienerseite (B) und/oder zu der Patientenseite (P) hin gewölbt ist.

10. Schutzanordnung nach einem der vorhergehenden Ansprüche, **dadurch gekennzeichnet, dass** das Schutzflächenteil (1, 1a, 1b) im Bereich einer Unterseite mit einer nach unten offenen ergonomischen Durchgriffsöffnung (18, 18a, 18b) versehen ist, die ein Hindurchgreifen von der Bedienerseite (B) zu der Patientenseite (P) ermöglicht.

11. Schutzanordnung nach einem der vorhergehenden Ansprüche, **dadurch gekennzeichnet, dass** dem Schutzflächenteil (1, 1a, 1b) eine formschlüssig wirksame Verdrehsicherung zugeordnet ist, die in montiertem Zustand des Schutzflächenteils (1, 1a) eine verdrehsichere Fixierung auf dem Aufnahmeabschnitt ermöglicht.

12. Schutzanordnung nach einem der vorhergehenden Ansprüche, **dadurch gekennzeichnet, dass** das Schutzflächenteil (1) patientenseitig wenigstens eine optisch signifikante Markierung (13) aufweist, die eine Patientin/ein Patient optisch fokussieren kann.

13. Schutzanordnung nach einem der vorhergehenden Ansprüche, **dadurch gekennzeichnet, dass** dem Schutzflächenteil (1b) eine Absaugeinrichtung zugeordnet ist, die zur Absaugung von Aerosolen im Bereich des Schutzflächenteils (1b) ausgebildet ist.

14. Schutzanordnung nach Anspruch 13, **dadurch gekennzeichnet, dass** die Absaugeinrichtung wenigstens eine entlang des Schutzflächenteils (1b) verlegte Schlauch- oder Rohreinheit (16) aufweist, der ein Saugantrieb (17) zugeordnet ist.

15. Schutzanordnung nach Anspruch 14, **dadurch gekennzeichnet, dass** die Schlauch- oder Rohreinheit (16) mehrere über eine Länge der Schlauch- oder Rohreinheit (16) verteilt angeordnete Saugöffnungen (19) aufweist.
